# EUROPEAN PATENT APPLICATION

(11) **EP 4 023 253 A1**
(43) Date of publication of application: **06.07.2022**
(21) Application number: 21210155.4
(22) Date of filing: 24.11.2021
(51) Int. Cl.: A61L 2/10, B08B 7/00, A61B 5/26

(54) **MEDICAL DEVICE WITH HANDLE STERILIZATION**

(30) Priority: 31.12.2020 TR 202022623
(71) Applicant: Arçelik Anonim Sirketi, 34445 Istanbul (TR)
(72) Inventor: GOODSPEED, Chiu, 34445 ISTANBUL (TR); LIN, Norman, 34445 ISTANBUL (TR); CHEN, Louis, 34445 Istanbul (TR)

(57) **Abstract**

The present invention relates to a medical device (1) for measuring physiological parameters from hand of a patient. The medical device comprises a handle (2) and an electrode (3) disposed on the handle (2) for taking electrical measurements from the hand.

## Description

The present invention relates a medical device for measuring physiological parameters such as electrocardiogram waveforms from a hand of a patient.

Physiological measurements such as electrocardiography (ECG) measurements from hands of a patient can be done by means of medical devices comprising handles with electrodes. These devices help people conveniently monitor their heart functioning, levels of fat burning, and/or diagnose potential problems regarding heart rhythm disorders. Given that handles of these devices are touched at each use and in some cases by different patients, proper sterilization of handles is critical for preventing spread of germs. Germs and other residues accumulating on the handle, especially on and around the electrodes also adversely affect accuracy of measurements. Accordingly, there is need for a medical device wherein sterilization of handles is realized in a simple and efficient manner.

In the state of the art there are examples using ultraviolet (UV) germicidal lamps for sterilization of frequently touched surfaces, such as door handles and knobs.

United States patent application US2020217102A1 discloses an antibacterial doorknob. The doorknob is made of transparent material and a UV light source is concealed therein for sterilizing the surface of the doorknob by UV radiation.

European patent application EP2314802A2 discloses a rotary handle comprising a UV radiation source for disinfection of the handle. The UV radiation source is arranged in a cylindrical casing around the rotary handle.

The aim of the present invention is the realization of a medical device for measuring physiological parameters from hand of a patient, wherein the handle of the device is efficiently sterilized in a simple and cost-effective way.

The invention proposes a medical device wherein handle or handles of the device can be sterilized and disinfected by ultraviolet irradiation means comprised therein. Sterilization of the handle in this way helps improving accuracy of measurements by removing residual layers on the handle and obviating the need for using additional cleaning agents such as disinfecting liquids.

The aim of the present invention is achieved by the medical device defined in claim 1. Further achievements have been attained by the subject-matters respectively defined in the dependent claims.

The medical device for measuring physiological parameters from a hand of a patient comprises a handle, and an electrode disposed on the handle for taking electrical measurements from the hand.

In a preferred embodiment, the medical device further comprises an ultraviolet (UV) irradiation means disposed on the handle, for sterilizing the gripping surface of the handle. Accordingly, spread of germs is effectively prevented by removing wide range of bacteria, fungi, etc. and infection risk of user are minimized. By reducing breeding and distribution of bacteria and other residues on the handle surface, possible distorting effect of germs on the handle is prevented. By obviating the need for applying external disinfection and cleaning means, such as liquids or gels, on the gripping surface of the handle, possible distorting effects of these agents on measurement data is also avoided. UV germicide irradiation is also advantageous in that it has no side effects to users, causes no damage or corrosion on the handle surface and leaves no residues or pollutive traces after use.

Ultraviolet irradiation means may comprise at least one UVC lamp or at least one UVC light emitting diode (LED). Preferably, UV irradiation means comprises a plurality of UVC light emitting diodes (LEDs). UVC LEDs have a relatively longer lifetime and they are easy and cost-effective to apply, replace and maintain. LEDs are also advantageous due to stable and uniform irradiation they provide. Given that LEDs contain no infrared spectrum, they do not induce additional heating on handle surface, which could otherwise adversely affect accuracy of measurements. Another benefit of using LEDs is that there is almost no warm-up time needed for a LED when activated. Sterilization process can be completed more quickly, getting the medical device clean and ready for next use in a relatively shorter time.

The medical device may further comprise a carrier on the handle for accommodating the UV irradiation means. The carrier may be position on an end of the handle, for example, at or close to a distal end of the handle. The carrier preferably comprises a mounting surface for mounting the UV irradiation means, extending around circumference of the handle. In this way, the carrier does not take up or interfere with the gripping surface area. Furthermore, irradiation means can be positioned at one fixed portion on the handle to irradiate the whole gripping surface. To this end, the UV irradiation means can be arranged on the carrier to face the gripping surface of the handle. The UV irradiation means is preferably arranged on the carrier such that, when activated, the UV irradiation means irradiates the entire gripping surface of the handle. As the working area of the irradiation means covers the whole gripping surface where a user's hand typically contacts, the whole contact surface potentially bearing germs can be effectively sterilized.

According to an embodiment, the measured physiological parameters relate to heartbeat induced pulsations. The measured physiological parameters may comprise an electrocardiogram (ECG) waveform.

In at least one embodiment of the invention, the medical device further comprises a power control means for activating and deactivating the UV irradiation means. By using the power control means, the handle can be easily sterilized before and/or after each use of the device to reduce bacterial adhesion and residue to prevent spread of germs. The medical device may further comprise a detection means for detecting presence of a hand on the handle. The power control means may be arranged to activate the UV irradiation means only when no hand is detected on the handle by the detection means. The power control means may be arranged to automatically activate the UV irradiation means when the detection means detects that a hand is removed from the handle. Thus, the handle can be automatically sterilized and made ready for next use free of infection risks.

The medical device may be a hand-held device. Additionally, or alternatively, the medical device can be part of a health kiosk, a medical equipment system or a fitness equipment.

The medical device realized in order to attain the aim of the present invention is illustrated in the attached figure, where:

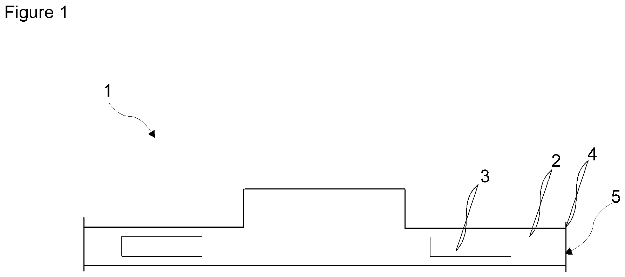

Figure 1 is a front perspective view of the medical device according to an embodiment.

The elements illustrated in the figure are numbered as follows:
1. Medical device
2. Handle
3. Electrode
4. Irradiation means
5. Carrier

The medical device (1) for measuring physiological parameters from hand of a patient comprises a handle (2) and an electrode (3) disposed on the handle (2) for taking electrical measurements from the hand.

In a preferred embodiment, the medical device (1) further comprises an ultraviolet (UV) irradiation means (4) disposed on the handle (2) for sterilizing the gripping surface of the handle (2).

In operation, the medical device (1) is electrically powered, for example, by means of a power chord from a power outlet or a battery (not shown in figures). The medical device (1) comprises or is coupled to at least one sensor. The signals detected by the electrode (3) based on heartbeat induced pulsations of the patient can be received by the sensor. The medical device (1) comprises or is coupled to a processing resource where sensed parameters are received from the at least one sensor and processed to obtain data relating to measured physiological parameters, such as ECG waveforms. Measured data may be transmitted to an external computing device and/or displayed on an interface, such as display means connected to the medical device (1).

The medical device (1) preferably comprises two handles (2), one for gripping by each hand. The handle (2) may be in the form of a handlebar, for example a cylindrical handlebar.

UVC germicidal lamps, preferably UVC LEDs are used as UV irradiation means. In a preferred embodiment of the invention, the ultraviolet irradiation means (4) comprises a plurality of UVC light emitting diodes (LEDs). (Figure 1)

According to at least one embodiment, the medical device (1) further comprises a carrier (5) on the handle (2) for accommodating the UV irradiation means (4). The carrier (5) is preferably a single-piece part fixed to the handle (2). In other words, the carrier (5) preferably does not include modular or moving parts. The carrier (5) is disposed on an end of the handle (2), for example at a position at or in proximity of a distal edge of the handle (2). The carrier (5) comprises at least one mounting surface for mounting the UV irradiation means (4). The mounting surface extends around circumference of the handle (2). The carrier (5) may be in the shape of a circular plate having a radius larger than the handle (2), fixed to a distal end of the handle (2). Alternatively, the carrier (5) may be a ring/sleeve type portion disposed on an end of the handle (2). The mounting surface may therefore be an inner surface of a circumferential ring formed by the carrier (5). The mounting surface may comprise one or more recesses or holes for receiving the irradiation means (4).

The UV irradiation means (4), when mounted on the carrier (5), face the gripping surface of the handle (2). The UV irradiation means (4) is arranged on the carrier (5) such that, when activated, the UV irradiation means (4) irradiates the entire gripping surface of the handle (2). For example, a plurality of UVC LEDs may be arranged on the mounting surface on a circular path to irradiate the entire gripping surface.

In at least one embodiment of the invention, the measured physiological parameters comprise an electrocardiogram (ECG) waveform. The medical device (1) may be an ECG device, for example a heart-rate monitoring device.

The medical device (1) may further comprise a power control means for activating and deactivating the UV irradiation means (4). Power control means can be configured to turn the UV irradiation means (4) on/off manually and/or automatically. For example, the power control means, or an additional processing unit for controlling the power control means, may be configured to automatically activate the UV irradiation means (4) when medical device (1) is not in use for a certain time and/or when the medical device (1) goes into standby mode. Power control means may further be configured to activate UV irradiation means and sterilize the handle (2) according to a set routine, for example at certain times of day.

The medical device (1) may further comprise a detection means for detecting presence of a hand on the handle (2). The power control means may be arranged to activate the UV irradiation means (4) only when no hand is detected on the handle (2) by the detection means. In an embodiment, the power control means is arranged to activate the UV irradiation means (4) when the detection means detects that a hand is removed from the handle (2). Power control means may be arranged to activate the UV irradiation means (4) after a predetermined time, such as 30 seconds, 1 minute, or 5 minutes passes upon removal of hand from the handle (2).

The medical device (1) may a hand-held device, for example an ECG device for personal use. Additionally, or alternatively, the medical device (1) can be comprised in a health kiosk, a medical equipment system or a fitness equipment. The present invention thus extends to a health kiosk, a medical equipment system or a fitness equipment comprising the medical device (1).

In view of technical features and details explained above, the present invention proposes a medical device (1) wherein a handle (2) thereof can be effectively sterilized by UV irradiation means (4) provided on the handle (2). The medical device (1) helps preventing spread of germs and infections. Furthermore, when compared to common techniques used in sterilizing medical devices, sterilization time is minimized, and accuracy of measurements obtained by the medical device (1) is improved.

## Claims

1. A medical device (1) for measuring physiological parameters from hand of a patient, comprising a handle (2) and an electrode (3) disposed on the handle (2) for taking electrical measurements from the hand; **characterised in that** the medical device (1) further comprises an ultraviolet (UV) irradiation means (4) disposed on the handle (2) for sterilizing the gripping surface of the handle (2).

2. The medical device (1) according to claim 1, wherein ultraviolet irradiation means (4) comprises a plurality of UVC light emitting diodes (LEDs).

3. The medical device (1) according to claim 1 or 2, wherein the medical device (1) further comprises a carrier (5) on the handle (2) for accommodating the UV irradiation means (4).

4. The medical device (1) according to claim 3, wherein the carrier (5) is disposed on an end of the handle (2).

5. The medical device (1) according to claim 4, wherein the carrier (5) comprises a mounting surface for mounting the UV irradiation means (4), extending around circumference of the handle (2).

6. The medical device (1) according to any one of claims 3 to 5, wherein the UV irradiation means (4) on the carrier (5) face the gripping surface of the handle (2).

7. The medical device (1) according to claim 6, wherein the UV irradiation means (4) is arranged on the carrier (5) such that, when activated, the UV irradiation means (4) irradiates the entire gripping surface of the handle (2).

8. The medical device (1) according to any one of preceding claims, wherein the measured physiological parameters comprise an electrocardiogram (ECG) waveform.

9. The medical device (1) according to any one of preceding claims, wherein the medical device (1) further comprises a power control means for activating and deactivating the UV irradiation means (4).

10. The medical device (1) according to claim 9, wherein the medical device (1) further comprises a detection means for detecting presence of a hand on the handle (2).

11. The medical device (1) according to claim 10, wherein the power control means is arranged to activate the UV irradiation means (4) only when no hand is detected on the handle (2) by the detection means.

12. The medical device (1) according to claim 10 or 11, wherein the power control means is arranged to activate the UV irradiation means (4) when the detection means detects that a hand is removed from the handle (2).

13. The medical device (1) according to any one of preceding claims, wherein the medical device (1) is a hand-held device.
